# EUROPÄISCHE PATENTSCHRIFT

(11) **EP 0 860 427 B1**
(45) Veröffentlichungstag und Bekanntmachung des Hinweises auf die Patenterteilung: **05.09.2001**
(21) Anmeldenummer: 98103195.8
(22) Anmeldetag: 24.02.1998
(51) Int. Cl.: C07C 253/34, C07C 255/04, C07C 253/14, C07C 209/48

(54) **Verfahren zur Entfernung von Halogeniden aus halogenidhaltigen Nitrilgemischen**
Process for the removal of halides from halide containing nitrile mixtures
Procédé pour l'élimination d'halogénures de mélanges de nitriles contenant d'halogénures

(30) Priorität: 25.02.1997 DE 19707509
(43) Veröffentlichungstag der Anmeldung: 26.08.1998
(73) Patentinhaber: BASF AKTIENGESELLSCHAFT, 67056 Ludwigshafen (DE)
(72) Erfinder: Kneuper, Heinz-Josef, Dr., 68163 Mannheim (DE); Weyer, Hans-Jürgen, Dr., 67240 Bobenheim-Roxheim (DE); Neuhauser, Horst, Dr., 67373 Dudenhofen (DE); Melder, Johann-Peter, Dr., 67141 Neuhofen (DE); Henne, Andreas, Dr., 67433 Neustadt (DE); Ross, Karl-Heinz, Dr., 67269 Grünstadt (DE); Becker, Rainer, Dr., 67098 Bad Dürkheim (DE)
(74) Vertreter: Isenbruck, Günter, Dr.

(56) Entgegenhaltungen:
- DE-A- 2 211 060
- DE-B- 1 046 601
- DE-C- 920 789
- US-A- 2 570 794
- US-A- 2 695 912
- DATABASE WPI Week 8906 Derwent Publications Ltd., London, GB; AN 89-041527 XP002069400 & JP 63 196 550 A (MITSUBISHI GAS CHEM CO INC)
- DATABASE WPI Week 9125 Derwent Publications Ltd., London, GB; AN 91-183214 XP002069401 & JP 03 112 945 A (MITSUBISHI GAS CHEM CO INC)

## Beschreibung

Die Erfindung betrifft ein Verfahren zur Entfernung von Halogeniden aus halogenidhaltigen Nitrilgemischen sowie ein Verfahren zur Herstellung von Aminen aus diesen halogenidhaltigen Nitrilgemischen.

Aliphatische Amine werden häufig durch Hydrierung von Nitrilen hergestellt. Die Nitrile sind dabei nach der Kolbe-Nitrilsynthese durch Umsetzung von Alkylhalogeniden mit Metallcyaniden zugänglich. Ein entsprechendes Verfahren zur Herstellung von Alkylcyaniden ist beispielsweise in Organikum, organisch-chemisches Grundpraktikum, 16. Auflage, VEB Deutscher Verlag der Wissenschaften, Berlin 1986, Seiten 210 bis 213 beschrieben. Die Nitrile enthalten dabei herstellungsbedingt Halogenide, die sich insbesondere von als Phasentransferkatalysator verwendeten Tetraalkylammoniumhalogeniden ableiten, wenn die Herstellung der Nitrile in Gegenwart von Phasentransferkatalysatoren erfolgt. Die Halogenide führen dabei zu einer starken Korrosion der verwendeten Reaktoren. Zudem werden Festbettkatalysatoren durch die Halogenide desaktiviert, so daß nur eine batchweise Hydrierung möglich ist.

Um die Hydrierung kontinuierlich an Festphasenkatalysatoren durchführen zu können, ist die Entfernung der Halogenide aus den Nitrilen vor der Hydrierung notwendig.

US 2,695,912 betrifft die Isomerisierung von 1,4-Dicyano-2-buten. Im Verfahren wird das erhaltene rohe Dicyanobuten erhitzt und mit erhitzter Natronlauge versetzt, um chlorhaltige Verunreinigungen zu entfernen.

DE-A-2 211 060 betrifft ein Verfahren zur Reinigung von Dinitrilen. Dabei wird insbesondere 1,4-Dicyanbuten-2-trans durch Umsetzung von Dibrombutenen und Blausäure gewonnen. Das Produkt wird mit Toluol extrahiert und nach dem Abdestillieren des Toluols mit Hexamethylendiamin versetzt. Sodann wird das Dycianbuten vom Rückstand abdestilliert.

Aufgabe der vorliegenden Erfindung ist die Bereitstellung eines Verfahrens zur Entfernung von Halogeniden aus halogenidhaltigen Nitrilgemischen, das die anschließende Herstellung von Aminen aus den Nitrilgemischen erlaubt.

Gelöst wird die Aufgabe durch Bereitstellung eines Verfahrens zur Entfernung von Halogeniden aus halogenidhaltigen Nitrilgemischen wobei das Halogenid im halogenidhaltigen Nitrilgemisch ganz oder teilweise in Form eines Tetraalkylammoniumhalogenid-Phasentransferkatalysators vorliegt, durch
(a) thermische Behandlung des halogenidhaltigen Nitrilgemisches,
(b) nachfolgendes Versetzen des thermisch behandelten Nitrilgemisches mit einer Base und anschließend
(c) Abtrennen der Base aus dem Nitrilgemisch.

Es wurde gefunden, daß bei der Herstellung von Nitrilen, insbesondere Korksäuredinitril durch Umsetzung von 1,6-Dichlorhexan mit wäßriger Cyanidlösung in Gegenwart von Tetraalkylammoniumhalogeniden als Phasentransferkatalysator das erhaltene Nitril Halogenid in Mengen von etwa 500 bis 1000 ppm enthält. Das Halogenid hat Tetraalkylammoniumionen als Gegenionen und ist damit Bestandteil eines Phasentransferkatalysator. Dieser Phasentransferkatalysator kann naturgemäß nicht durch Wäsche mit Wasser oder Basen aus dem organischen Nitrilgemisch entfernt werden, da er sich sowohl in der wäßrigen als auch in der organischen Phase löst. Zur Abtrennung des Halogenids und zur weiteren Aufreinigung des Nitrils kann das rohe Nitril, insbesondere Korksäuredinitril, destilliert werden. Im Destillat wurden jedoch immer noch signifikante Mengen an Halogenid von etwa 400 bis 500 ppm gefunden, so daß die Abtrennung von Halogenid weder durch Waschen mit Wasser oder Basen noch durch Destillation zum Erfolg führt.

Erfindungsgemäß wurde gefunden, daß die Entfernung der Halogenide jedoch durch eine thermische Behandlung des rohen Nitrilgemisches und nachfolgendes Versetzen des thermisch behandelten Nitrilgemisches mit einer Base möglich ist. Ohne an die folgende Vorstellung über den Reaktionsablauf gebunden zu sein, wird angenommen, daß Tetraalkylammoniumsalze bei thermischer Behandlung unter Abspaltung von Olefinen Trialkylammoniumhydrohalogenide bilden. Diese sind im Gegensatz zu Tetraalkylammoniumsalzen flüchtig, da sie unter thermischer Belastung in Trialkylamin und Halogenwasserstoff dissoziieren. Diese beiden Komponenten sind gasförmig beziehungsweise verdampfbar und können eine Destillation passieren, um bei der Kondensation wieder Trialkylarnmoniumhydrohalogenid zu bilden.

Die nach der thermischen Behandlung vorliegenden Trialkylammoniumhydrohalogenide können jedoch durch Umsetzung mit einer Base und anschließendes Abtrennen der Base aus dem Nitrilgemisch entfernt werden. Durch das erfindungsgemäße Verfahren kann der Gehalt an Halogenid auf einen sehr kleinen Wert, vorzugsweise unter 10 ppm (Nachweisgrenze) vermindert werden. Die so erhaltenen Nitrilgemische bzw. Nitrile können katalytisch hydriert werden, ohne daß die verwendeten Apparaturen Korrosion zeigen oder die Katalysatoren desaktiviert werden.

Die thermische Behandlung in Stufe (a) wird so durchgeführt, daß im halogenidhaltigen Nitrilgemisch vorliegende Tetraalkylammoniumhalogenide in Trialkylammoniumhydrohalogenide umgewandelt werden. Vorzugsweise erfolgt die thermische Behandlung bei einer Temperatur von 50 bis 350°C, besonders bevorzugt 150 bis 300°C für einen Zeitraum, der ausreicht zur Umwandlung von Tetraalkylammoniumhalogeniden in Trialkylammoniumhydrohalogenide. Vorzugsweise ist die thermische Behandlung eine Destillation bei einem Druck und bei einer Temperatur, bei denen das im Gemisch enthaltene Nitril destilliert wird. Beispielsweise wird eine kontinuierliche Destillation von Korksäuredinitril bei einem Druck am Destillationskopf von 50 mbar, einer Sumpftemperatur von 227 bis 230°C und einer Kopftemperatur von 163°C durchgeführt. Andere Möglichkeiten der thermischen Behandlung sind dem Fachmann bekannt.

Nach der thermischen Behandlung wird das thermisch behandelte Nitrilgemisch mit einer Base versetzt. Als Base werden beispielsweise Alkalimetallhydroxide, -hydrogencarbonate oder -carbonate verwendet, insbesondere in Form wäßriger Lösungen. Besonders bevorzugt werden die Natrium- oder Kaliumverbindungen eingesetzt, wobei die Konzentration an Alkalihydroxid in der wäßrigen Alkalimetallhydroxidlösung vorzugsweise 5 bis 50, insbesondere 10 bis 30 Gew.-% beträgt. Wäßrige Lösungen von Alkalimetallhydrogencarbonaten oder Alkalimetallcarbonaten sind vorzugsweise konzentriert, insbesondere gesättigt. Das Versetzen des thermisch behandelten Nitrilgemisches mit der wäßrigen Basenlösung erfolgt vorzugsweise unter Rühren, um einen guten Kontakt der organischen mit der wäßrigen Phase zu gewährleisten. Dabei werden Trialkylammoniumhydrohalogenide unter Bildung von nur wasserlöslichem Alkalimetallhalogenid und Trialkylaminen deprotoniert. Das Alkalimetallhalogenid ist dabei weitestgehend in der wäßrigen Phase enthalten, so daß der Halogenidgehalt in der organischen Nitrilgemischphase vorzugsweise auf unter 10 ppm sinkt.

Insbesondere bei Verwendung konzentrierter Basenlösungen erfolgt die Phasentrennung in organische Nitrilgemischphase und wäßrige Phase leicht und nahezu vollständig. Die Absitzzeiten, d.h. die zur Phasentrennung benötigten Zeiten, liegen vorzugsweise im Minutenbereich. Dabei werden vorzugsweise mehr als 80%, besonders bevorzugt mehr als 90%, insbesondere 95% oder mehr der wäßrigen Lösung zurückgewonnen. Das Versetzen mit der wäßrigen Basenlösung erfolgt bei einer Temperatur, bei der eine Nitrilverseifung vollständig oder weitgehend verhindert wird. Vorzugsweise wird bei Umgebungstemperatur gearbeitet. Es wurde hierbei keine Nitrilverseifung beobachtet, die sich in einem Anstieg des Destillationsrückstandes bei einer nachfolgenden Destillation des Nitrilgemisches zeigen würde.

Als Base kann auch eine in festem Zustand vorliegende Base, beispielsweise ein basischer Ionenaustauscher verwendet werden. Die Abtrennung des Ionenaustauschers aus dem Nitrilgemisch ist dabei einfach, beispielsweise durch Dekantieren oder Filtrieren, möglich.

Vorzugsweise wird das Versetzen mit einer Base in Stufe (b) mit Natriumhydroxid, Natriumhydrogencarbonat oder Natriumcarbonat oder entsprechenden Kaliumsalzen in Form einer Lösung oder in fester Form durchgeführt.

Die so erhaltenen gereinigten Nitrile, beispielsweise Korksäuredinitril, können an Suspensions- oder insbesondere an Festbettkatalysatoren hydriert werden, ohne daß es zu einer Korrosion im Reaktor oder zu einer Desaktivierung des Katalysators kommt. Die Erfindung betrifft auch ein Verfahren zur Herstellung von Aminen aus halogenidhaltigen Nitrilgemischen durch Entfernung von Halogeniden aus den halogenidhaltigen Nitrilgemischen gemäß der vorstehend beschriebenen Stufen (a), (b) und (c) und nachfolgendes Hydrieren von in Stufe (c) erhaltenen Nitrilen zu Aminen in Gegenwart von Suspensions- oder Festbettkatalysatoren (Stufe (d)). Als Suspensions- oder Festbettkatalysatoren können alle geeigneten Katalysatoren eingesetzt werden. Vorzugsweise werden die in DE-A-44 46 893 beschriebenen Katalysatoren verwendet. Insbesondere enthält der zur Hydrierung verwendete Katalysator Nickel und/oder Cobalt und/oder Palladium und/oder Platin und/oder Eisen und/oder Ruthenium. Besonders bevorzugt enthält der Katalysator 90 Gew.-% CoO, 5 Gew.-% Mn₂O₃, 3 Gew.-% P₂O₅ und 2 Gew.-% Na₂O.

Geeignete Reaktionsbedingungen für die Hydrierung sind beispielsweise in DE-A-44 46 893 beschrieben. Die Temperatur beträgt vorzugsweise 60 bis 160°C, besonders bevorzugt 90 bis 130°C, der Gesamtdruck vorzugsweise 20 bis 300 bar, besonders bevorzugt 150 bis 250 bar, das Molverhältnis von Nitrilgruppen zu Ammoniak vorzugsweise 1:1 bis 1:50, besonders bevorzugt 1:5 bis 1:20.

Die Hydrierung kann dabei kontinuierlich oder diskontinuierlich durchgeführt werden. Vorzugsweise wird sie kontinuierlich durchgeführt, insbesondere in Gegenwart eines Festbettkatalysators. Da zur Hydrierung der erfindungsgemäß gereinigten Nitrilgemische auch Festbettkatalysatoren ohne Gefahr einer Desaktivierung verwendet werden können, kann die Hydrierung kontinuierlich, und somit wesentlich ökonomischer durchgeführt werden, als es bisher möglich war.

Die im erfindungsgemäßen Verfahren eingesetzten halogenidhaltigen Nitrilgemische stammen vorzugsweise aus der Umsetzung von Alkylhalogeniden mit Metallcyaniden. Die Erfindung betrifft auch ein Verfahren zur Herstellung von Aminen durch
(A) Umsetzung von Alkylhalogeniden mit Alkalimetallcyaniden in einem mindestens zweiphasigen Reaktionsmedium in Gegenwart von Tetraalkylammoniumhalogenid-Phasentransferkatalysatoren zu Alkylnitrilen,
(B) Abtrennung der erhaltenen halogenidhaltigen Alkylnitrilgemisch-Phase und
(C) Weiterbehandlung der halogenidhaltigen Alkylnitrilgemisch-Phase gemäß den Stufen (a) bis (d), wie sie vorstehend beschrieben sind.

Die Umsetzung von Alkylhalogeniden mit Metallcyaniden zu Nitrilen ist allgemein als Kolbe-Nitrilsynthese bekannt. Dabei werden vorzugsweise primäre oder sekundäre, insbesondere primäre Alkylhalogenide eingesetzt. Die Alkylhalogenide basieren dabei vorzugsweise auf einem aliphatischen C₁₋₃₀-, vorzugsweise C₁₋₂₀-, besonders bevorzugt C₂₋₁₈-, insbesondere C₄₋₁₀-Rest, der durch 1 bis 5 Halogenatome substituiert ist und durch C₁₋₆-Alkyl, C₆₋₁₀-Aryl, C₁₋₆-Alkoxy, C₆₋₁₀-Aryloxy, COOH, OH substituiert oder als C₂₋₃₀-, vorzugsweise C₂₋₂₀-, besonders bevorzugt C₂₋₁₈, insbesondere C₄₋₁₀-Rest durch C₆₋₂₀-Aryl unterbrochen sein kann. Vorzugsweise ist der Rest durch 1 bis 3, insbesondere 1 oder 2 Halogenatome substituiert. Die Halogenatome sind dabei vorzugsweise Chlor- oder Brom-, insbesondere Chloratome. Vorzugsweise liegen keiner oder ein bis drei der genannten weiteren Substituenten vor. Beispiele geeigneter Substituenten sind Methyl, Ethyl, Propyl, Butyl, Pentyl, Hexyl in deren strukturisomere Formen, Phenyl, Naphthyl, Methoxy, Ethoxy, Propoxy, Butoxy, Pentoxy, Hexoxy, Phenyloxy, Naphthyloxy. Ein mit Phenyl oder Naphthyl substituierter aliphatischer C₁₋₂₀-Rest ist vorzugsweise ein Methylrest, so daß eine Benzyl- oder Methylnaphthalin-Struktur resultiert. Die Arylreste, wie Phenyl oder Naphthyl können dabei zusätzlich durch die vorstehenden Reste, vorzugsweise Alkoxy- und Alkylreste, insbesondere C₁₋₆-, speziell C₁₋₂-Alkoxy- oder Alkylreste substituiert sein. Beispiele von durch C₆₋₂₀-Aryl unterbrochene aliphatische C₂₋ ₂₀-Reste sind o-, m-, p-Dimethylbenzol oder entsprechende Diethyl- oder Dipropylverbindungen.

Vorzugsweise ist der Rest ein linearer aliphatischer C₁₋₂₀-Rest, der endständig durch jeweils ein Halogenatom substituiert ist und keine weiteren Substituenten aufweist. Dabei ist der Rest vorzugsweise ein linearer aliphatischer C₂₋₁₈-, insbesondere C₄₋₁₀-Rest.

Das Nitril basiert auf einem aliphatischen C₁₋₃₀-Rest, der durch 1 bis 5 Cyanogruppen substituiert ist und durch C₁₋₆-Alkyl, C₆₋₁₀-Aryl, C₁₋₆-Alkoxy, C₆₋₁₀-Aryloxy, COOH, OH substituiert oder als C₂₋₃₀-Rest durch C₆₋₁₀-Aryl unterbrochen sein Kann.

Die Umsetzung erfolgt mit Metallcyaniden, vorzugsweise Alkalimetallcyaniden, insbesondere Natriumcyanid. Das mindestens zweiphasige Reaktionsmedium weist dabei vorzugsweise eine wäßrige Phase und eine Alkylhalogenid enthaltende organische Phase auf, es handelt sich somit um ein zweiphasiges Reaktionsmedium. Die organische Phase kann dabei zusätzlich mindestens ein weiteres organisches Lösungsmittel enthalten, das an der Reaktion nicht teilnimmt.

Die Umsetzung wird dabei in Gegenwart von Tetraalkylammoniumhalogeniden ausgeführt. Dabei liegt mindestens ein C₁₋₃₀-, vorzugsweise C₁₋₂₀-Alkylrest vor, vorzugsweise liegen mindestens 2, besonders bevorzugt 3 oder 4 dieser Alkylreste vor. Dabei können ein C₈₋₂₄-Alkylrest und ein bis drei C₁₋₈-Alkylreste vorliegen. Die Alkylreste, insbesondere Methylreste können mit aromatischen Resten substituiert sein. So können Phenylreste über Alkylenreste an das Stickstoffatom gebunden sein. Beispiele derartiger Verbindungen sind Benzyltrialkylammoniumhalogenide, wie Benzyl-C₁₂₋₁₈-Alkyldimethylammoniumhalogenide.

Besonders bevorzugt werden Tetra-C₁₋₁₀-, insbesondere Tetra-C₃₋₈-alkylammoniumhalogenide eingesetzt. Besonders bevorzugt sind die Chloride und Bromide.

Die erhaltenen halogenidhaltigen Alkylnitrilgemische enthalten Alkylnitrile, die auf den vorstehend beschriebenen Alkylhalogeniden basieren, mit dem Unterschied, daß die Halogenatome durch Cyanogruppen ersetzt sind. Die im erfindungsgemäßen Verfahren einsetzbaren Nitrilgemische enthalten somit die vorstehend aufgeführten Verbindungen, wobei die Halogenatome durch Cyanogruppen ersetzt sind.

Beispiele sind Dicyanopropan, -butan, -pentan, -hexan, -heptan, -octan,nonan, -decan, o/m/p-Alkoxyphenylacetonitril, Dialkoxyphenylacetonitril, Trialkoxyphenylacetonitril.

Die Bedingungen bei der Umsetzung der Alkylhalogenide mit Metallcyaniden sind dabei bekannt.

Die Abtrennung der bei der Umsetzung erhaltenen halogenidhaltigen Alkylnitrilgemisch-Phase (Stufe B) erfolgt vorzugsweise durch Phasentrennung des zweiphasigen Reaktionsmediums, gegebenenfalls gefolgt vom Abdestillieren einer in der organischen Phase verbliebenen wäßrigen Phase.

Ein erfindungsgemäßes Verfahren zur Herstellung von Aminen, insbesondere aliphatischen Monoaminen oder aliphatischen Diaminen erfolgt durch Umsetzung von Alkylhalogeniden mit Metallcyaniden gemäß Stufe (A), Abtrennung der erhaltenen halogenidhaltigen Alkylnitrilgemisch-Phase in Stufe (B) durch Phasentrennung und anschließendes Andestillieren, wobei Reste der in der organischen Phase verbliebenen wäßrigen Phase entfernt werden, Destillieren des halogenidhaltigen Alkylnitrilgemisches in Stufe (a), Versetzen des thermisch behandelten Nitrilgemisches mit einer wäßrigen Baselösung in Stufe (b), Abtrennen der Base aus dem Nitrilgemisch durch Phasentrennung in Stufe (c), Hydrierung der in Stufe (c) erhaltenen Nitrile zu Aminen in Gegenwart von Festbettkatalysatoren und nachfolgende Destillation der erhaltenen Amine zu ihrer Reinigung.

Die Erfindung wird nachstehend anhand von Beispielen näher erläutert.

### Beispiele

### BEISPIEL 1

### Herstellung von Korksäuredinitril

Korksäuredinitril wurde aus 1,6-Dichlorhexan und wäßrigem NaCN (Molverhältnis 1:2,02) bei 100°C in Gegenwart von n-Bu₄NBr (3,2 Gew.-%, bezogen auf die eingesetzte Menge an 1,6-Dichlorhexan) kontinuierlich in einer Rührkesselkaskade hergestellt. Nach der Synthese wurden die Phasen getrennt. Die wäßrige Phase enthielt noch etwa 2 Gew.-% NaCN und wurde vor der Entsorgung mit Formaldehyd behandelt. Die organische Phase wurde batchweise 4 bis 5 mal mit Wasser gewaschen. Anschließend wurde zur Abtrennung von in der organischen Phase verbliebenem Wasser andestilliert.

Sodann wurde das Korksäuredinitrilgemisch in einer kontinuierlichen Destillation bei 50 mbar Druck am Kopf, 227 bis 230°C Sumpftemperatur und 163°C Kopftemperatur destilliert. Das so gereinigte Korksäuredinitril enthielt noch etwa 440 ppm Chlorid. In den nachfolgenden Untersuchungen wurde dieses destillierte Korksäuredinitril eingesetzt.

### BEISPIEL 2

### Reinigung von Korksäuredinitril

200 g Korksäuredinitril, das wie in Beispiel 1 beschrieben erhalten wurde, 700 g der nachstehenden wäßrigen Lösungen von NaOH, Na₂CO₃, NaHCO₃ oder VE-Wasser wurden für 0,5 bzw. 24 h gerührt. Sodann wurde eine Phasentrennung durchgeführt, wobei die Absitzzeit bestimmt wurde. Es wurden der Anteil der zurückgewonnenen wäßrigen Phase und der bei einer nachfolgenden Destillation erhaltene Destillationsrückstand bestimmt. Dieser Rückstand zeigt an, ob Korksäuredinitril durch die Extraktion hydrolysiert worden ist. Die Ergebnisse sind in der nachstehenden Tabelle 1 angegeben.

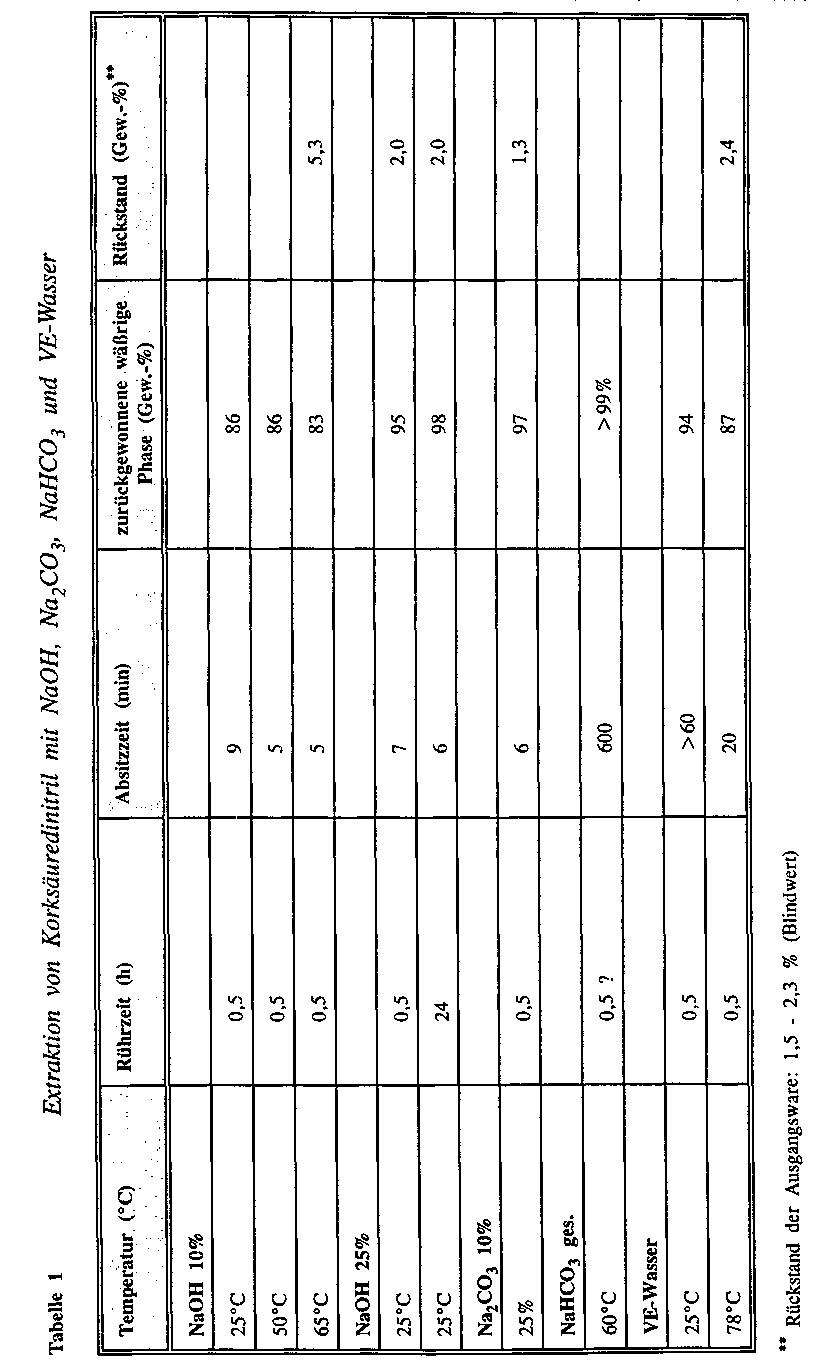

Der nach der Extraktion bestimmte Chloridgehalt betrug bei Verwendung von NaOH, Na₂CO₃ und NaHCO₃ weniger als 10 ppm. Die Verwendung von VE-Wasser ist in Beispiel 3 beschrieben.

Durch Verwendung der vorstehenden Basenlösungen konnte die Absitzzeit gegenüber der Verwendung von VE-Wasser deutlich verringert werden.

### BEISPIEL 3

### Reinigung von Korksäuredinitril

60 kg Korksäuredinitril (Chloridgehalt 440 ppm) aus Beispiel 1 wurden bei 60°C mit 20 kg Wasser ausgerührt (1h). Nach 24-stündigem Absitzen wurden 17 kg Wasser abgetrennt. Die organische Phase enthielt 70 ppm Chlorid. Anschließend wurden weitere 60 kg Korksäuredinitril mit 20 kg Wasser 1 h lang ausgerührt und nach 18 h die Phasen getrennt. Die organische Phase wurde mit 20 kg ges. NaHCO₃-Lösung versetzt und 1 h ausgerührt. Nach Abschalten des Rührers wurde 10 h absitzen lassen und 20 kg wäßrige Phase wurden abgetrennt und verworfen. Chloridgehalt: < 10 ppm. Die beiden organischen Phasen wurden vereinigt (Chloridgehalt 30 ppm) und in einem Hochdruckreaktor hydriert.

### BEISPIEL 4

### Reinigung von Korksäuredinitril

Zu 2000 kg 25%iger Natronlauge wurden unter Rühren bei 25°C 5000 kg Korksäuredinitril aus Beispiel 1 gepumpt. Anschließend wurde 5 h bei 25°C gerührt. Nach ca. 5-stündigem Absitzen und anschließender Trennung der Phasen wurde die untere wäßrige Phase abgelassen und verworfen und die obere organische Phase zur Weiterverarbeitung abgegeben.

### BEISPIEL 5

### Kontinuierliche Hydrierung von mit NaHCO₃ extrahiertem Korksäuredinitril

In einem 1 Liter fassenden Reaktor, gefüllt mit 0,5 1 eines Festbett-Kobaltkatalysators wurden bei 90°C und 200 bar Wasserstoffdruck 500 ml/h Ammoniak und 100 ml/h Korksäuredinitril kontinuierlich zugefahren. Es wurde wie in Beispiel 3 erhaltenes Korksäuredinitril eingesetzt, das einen Chloridgehalt von 30 ppm aufwies. Die Hochdruck-Hydrierung lief bei 100°C und einer Katalysatorbelastung von 0,2 l/l h über 21 Tage mit mehr als 99% Umsatz und 98% Selektivität. Eine Katalysatordesaktivierung konnte nicht beobachtet werden.

### BEISPIEL 6

### Kontinuierliche Hydrierung von mit NaOH extrahiertem Korksäuredinitril

In einem Hochdruckreaktor, der mit 60 l Katalysator gefüllt war, wurde Korksäuredinitril wie in Beispiel 3 beschrieben hydriert. Das Korksäuredinitril wurde jedoch mit 10%iger NaOH-Lösung chloridfrei (weniger als 10 ppm, Gehalt vorher 370 ppm) gewaschen. Die Hochdruck-Hydrierung lief bei 110°C und einer Katalysatorbelastung von 0,27 l/l h über 21 Tage mit mehr als 99% Umsatz und 98% Selektivität. Eine Katalysatordesaktivierung konnte nicht beobachtet werden.

### VERGLEICHSBEISPIEL

### Kontinuierliche Hydrierung von halogenhaltigem Korksäuredinitril

Das Verfahren gemäß Beispiel 5 wurde wiederholt, jedoch wurden 100 ml/h Korksäuredinitril (Chloridgehalt etwa 500 ppm) kontinuierlich zugefahren. Nach 1 Stunde Betriebsdauer waren 98% des Eduktes zum Produkt Diaminooctan umgesetzt. Nach 24 h war der Umsatz auf 11% zurückgefallen.

## Patentansprüche

1. Verfahren zur Entfernung von Halogeniden aus halogenidhaltigen Nitrilgemischen, wobei das Halogenid im halogenidhaltigen Nitrilgemisch ganz oder teilweise in Form eines Tetraalkylammoniumhalogenid-Phasentransferkatalysators vorliegt, durch
a) thermische Behandlung des halogenidhaltigen Nitrilgemisches,
b) nachfolgendes Versetzen des thermisch behandelten Nitrilgemisches mit einer Base und anschließend
c) Abtrennen der Base aus dem Nitrilgemisch.

2. Verfahren nach Anspruch 1, wobei die thermische Behandlung eine Destillation ist.

3. Verfahren nach Anspruch 1 oder 2, wobei als Base Alkalimetallhydroxide,-hydrogencarbonate oder -carbonate in Form einer wäßrigen Lösung oder in fester Form eingesetzt werden.

4. Verfahren nach einem der Ansprüche 1 bis 3, wobei die Base ein basischer Ionenaustauscher ist.

5. Verfahren nach einem der Ansprüche 1 bis 4, wobei das Nitril auf einem aliphatischen C₁₋₃₀-Rest basiert, der durch 1 bis 5 Cyanogruppen substituiert ist und durch C₁₋₆-Alkyl, C₆₋₁₀-Aryl, C₁₋₆-Alkoxy, C₆₋₁₀-Aryloxy, COOH, OH substituiert oder als C₂₋₃₀-Rest durch C₆₋₁₀-Aryl unterbrochen sein kann.

6. Verfahren nach Anspruch 5, wobei das Nitril ein Dinitril ist, das auf einem linearen aliphatischen C₁₋₂₀-Rest basiert, der endständig durch jeweils eine Cyanogruppe substituiert ist.

7. Verfahren zur Herstellung von Aminen aus halogenidhaltigen Nitrilgemischen, wobei das Halogenid im halogenidhaltigen Nitrilgemisch ganz oder teilweise in Form eines Tetraalkylammoniumhalogenid-Phasentransferkatalysators vorliegt, durch
(a) - (c) Entfernung von Halogeniden aus halogenidhaltigen Nitrilgemischen gemäß einem der Ansprüche 1 bis 6 und
(d) Hydrieren von in Stufe (c) erhaltenen Nitrilen zu Aminen in Gegenwart von Suspensions- oder Festbettkatalysatoren.

8. Verfahren zur Herstellung von Aminen durch
(A) Umsetzung von Alkylhalogeniden mit Metallcyaniden in einem mindestens zweiphasigen Reaktionsmedium in Gegenwart von Tetraalkylammoniumhalogenid-Phasentransferkatalysatoren zu Alkylnitrilen,
(B) Abtrennung der erhaltenen halogenidhaltigen Alkylnitrilgemisch-Phase und
(C) Weiterbehandlung der halogenidhaltigen Alkylnitrilgemisch-Phase gemäß den Stufen (a) bis (d) nach Anspruch 7.

## Claims

1. A process for removing halides from halide-containing nitrile mixtures wherein the halide in the halide-containing nitrile mixture is present in whole or in part in the form of a tetraalkylammonium halide phase-transfer catalyst by
(a) thermally treating the halide-containing nitrile mixture,
(b) subsequently adding a base to the thermally treated nitrile mixture and then
(c) separating off the base from the nitrile mixture.

2. A process as claimed in claim 1, wherein the thermal treatment is a distillation.

3. A process as claimed in claim 1 or 2, wherein, as base, use is made of alkali metal hydroxides, alkali metal hydrogen carbonates or alkali metal carbonates in the form of an aqueous solution or in solid form.

4. A process as claimed in one of claims 1 to 3, wherein the base is a basic ion exchanger.

5. A process as claimed in one of claims 1 to 4, wherein the nitrile is based on an aliphatic C₁₋₃₀ radical which is substituted by 1 to 5 cyano groups and can be substituted by C₁₋₆alkyl, C₆₋₁₀aryl, C₁₋₆alkoxy, C₆₋₁₀aryloxy, COOH, OH or, as C₂₋₃₀ radical, can be interrupted by C₆₋₁₀aryl.

6. A process as claimed in claim 5, wherein the nitrile is a dinitrile which is based on a linear aliphatic C₁₋₂₀ radical which is substituted by a cyano group at each end.

7. A process for preparing amines from halide-containing nitrile mixtures wherein the halide in the halide-containing nitrile mixture is present in whole or in part in the form of a tetraalkylammonium halide phase-transfer catalyst by
(a) - (c) removing halides from halide-containing nitrile mixtures according to one of claims 1 to 6 and
(d) hydrogenating nitriles obtained in stage (c) to give amines, in the presence of suspended or fixed-bed catalysts.

8. A process for preparing amines by
(A) reacting alkyl halides with metal cyanides in an at least two-phase reaction medium in the presence of tetraalkylammonium halide phase-transfer catalysts to give alkanenitriles,
(B) separating off the resulting halide-containing alkanenitrile mixture phase and
(C) further treating the halide-containing alkanenitrile mixture phase as described in the stages (a) to (d), as claimed in claim 7.

## Revendications

1. Procédé d'élimination d'halogénures à partir de mélanges à base de nitrile contenant de l'halogénure, dans lequel l'halogénure se présente dans le mélange à base de nitrile contenant de l'halogénure totalement ou partiellement sous la forme d'un catalyseur de transfert de phases à halogénure de tétraalkyl-ammonium, par
a) traitement thermique du mélange à base de nitrile contenant de l'halogénure,
b) addition ultérieure d'une base au mélange à base de nitrile thermiquement traité, et ensuite
c) séparation de la base à partir du mélange à base de nitrile.

2. Procédé suivant la revendication 1, dans lequel le traitement thermique est une distillation.

3. Procédé suivant l'une des revendications 1 et 2, dans lequel on met en oeuvre, comme base, des hydroxydes, bicarbonates ou carbonates de métal alcalin sous la forme d'une solution aqueuse ou sous forme solide.

4. Procédé suivant l'une des revendications 1 à 3, dans lequel la base est un échangeur d'ions basique.

5. Procédé suivant l'une des revendications 1 à 4, dans lequel le nitrile est basé sur un radical aliphatique en C₁-C₃₀ qui est substitué par 1 à 5 groupes cyano et qui peut être substitué par de l'alkyle en C₁-C₆, de l'aryle en C₆-C₁₀, de l'alcoxy en C₁-C₆, de l'aryloxy en C₆-C₁₀, COOH ou OH ou qui, en tant que radical en C₂-C₃₀, peut être interrompu par de l'aryle en C₆-C₁₀.

6. Procédé suivant la revendication 5, dans lequel le nitrile est un dinitrile qui est basé sur un radical aliphatique linéaire en C₁-C₂₀, qui est substitué en fin de chaîne chaque fois par un groupe cyano.

7. Procédé de préparation d'amines à partir de mélanges à base de nitrile contenant de l'halogénure, dans lequel l'halogénure se présente dans le mélange à base de nitrile contenant de l'halogénure totalement ou partiellement sous la forme d'un catalyseur de transfert de phases à halogénure de tétraalkyl-ammonium, par
(a)-(c) élimination d'halogénures à partir de mélanges à base de nitrile contenant de l'halogénure suivant l'une des revendications 1 à 6, et
(d) hydrogénation des nitriles obtenus dans l'étape (c) en amines, en présence de catalyseurs en suspension ou en lit fixe.

8. Procédé de préparation d'amines par
(A) réaction d'halogénures d'alkyle avec des cyanures de métal dans un milieu réactionnel à au moins deux phases, en présence de catalyseurs de transfert de phases à halogénure de tétraalkyl-ammonium, pour former des alkylnitriles,
(B) séparation de la phase de mélange à base d'alkylnitrile contenant de l'halogénure obtenue, et
(C) poursuite du traitement de la phase de mélange à base d'alkylnitrile contenant de l'halogénure selon les étapes (a) à (d) suivant la revendication 7.
